# EUROPÄISCHE PATENTSCHRIFT

(11) **EP 3 441 384 B1**
(45) Veröffentlichungstag und Bekanntmachung des Hinweises auf die Patenterteilung: **25.12.2019**
(21) Anmeldenummer: 17185366.6
(22) Anmeldetag: 08.08.2017
(51) Int. Cl.: C07C 67/38, C01B 32/39, C07C 69/00, C07C 69/02

(54) **METHOXYCARBONYLIERUNG MIT AMEISENSÄURE UND METHANOL**
METHOXYCARBONYLATION WITH FORMIC ACID AND METHANOL
MÉTHOXYCARBONYLATION AU MOYEN D'ACIDE FORMIQUE ET DE MÉTHANOL

(43) Veröffentlichungstag der Anmeldung: 13.02.2019
(73) Patentinhaber: Evonik Operations GmbH, 45128 Essen (DE)
(72) Erfinder: SANG, Rui, 252000 Liaocheng City, Shandong Province (CN); LIU, Jie, 412100 Zhuzhou (CN); DONG, Kaiwu, 236800 Bo Zhou (CN); JACKSTELL, Ralf, 10106 Rostock (DE); BELLER, Matthias, 18211 Ostseebad Nienhagen (DE); FRANKE, Robert, 45772 Marl (DE)
(74) Vertreter: Evonik Patent Association

(56) Entgegenhaltungen:
- EP-A2- 3 121 185
- KAIWU DONG ET AL: "Palladium-Catalyzed Carbonylation of sec - and tert -Alcohols", ANGEWANDTE CHEMIE INTERNATIONAL EDITION, Bd. 56, Nr. 22, 21. April 2017 (2017-04-21), Seiten 6203-6207, XP055426870, ISSN: 1433-7851, DOI: 10.1002/anie.201701950
- TSUMORU MORIMOTO ET AL: "Evolution of carbonylation catalysis: no need for carbon monoxide", ANGEWANDTE CHEMIE INTERNATIONAL EDITION, WILEY-VCH VERLAG GMBH , Bd. 43, Nr. 42 25. Oktober 2004 (2004-10-25), Seiten 5580-5588, XP002668525, ISSN: 1433-7851, DOI: 10.1002/ANIE.200301736 Gefunden im Internet: URL:http://onlinelibrary.wiley.com/doi/10. 1002/anie.200301736/abstract [gefunden am 2004-09-01]

## Beschreibung

Die Erfindung betrifft ein Verfahren zur Methoxycarbonylierung mit Ameisensäure und Methanol.

Die Methoxycarbonylierung von Alkenen ist ein Prozess mit steigender Bedeutung. Bei der klassischen Methoxycarbonylierung wird ein Olefin mit CO und MeOH in Gegenwart eines Katalysators, welcher einen Liganden und ein Metall umfasst, umgesetzt:

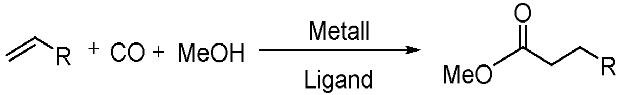

Hierbei wird CO als Gas in das Reaktionsgefäß eingeleitet (z.B. Angew. Chem. Int. Ed. 2017, 56, 6203).

Der Erfindung lag die Aufgabe zugrunde, ein Verfahren bereitzustellen, bei welchem eine andere CO-Quelle zum Einsatz kommt als CO-Gas, welches in das Reaktionsgefäß eingeleitet wird. Bei dem Verfahren sollte eine gute Ausbeute an Methylester erzielt werden.

Gelöst wird die Aufgabe durch ein Verfahren gemäß Anspruch 1.

Verfahren umfassend die Verfahrensschritte:
a) Zugabe eines Olefins;
b) Zugabe einer Verbindung, welche Pd umfasst, wobei das Pd in der Lage ist einen Komplex auszubilden;
c) Zugabe einer Verbindung der allgemeinen Formel (**I**): 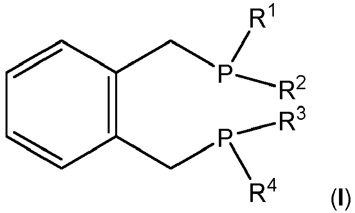 wobei R¹, R², R³, R⁴ jeweils unabhängig voneinander ausgewählt sind aus: -H, -(C₁-C₁₂)-Alkyl, - O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
   wobei die genannten Alkylgruppen, Arylgruppen, Cycloalkyl, Heteroalkylgruppen, Heteroarylgruppen wie folgt substituiert sein können: -(C₁-C₁₂)-Alkyl, -O-(C₁-C₁₂)-Alkyl, Halogen;
   und mindestens einer der Reste R¹, R², R³, R⁴ nicht für Phenyl steht;
d) Zugabe von MeOH;
e) Zugabe von HCOOH,
   wobei das eingesetzte Volumen bezogen auf 2 mmol Olefin im Bereich von 0,3 ml bis 0,8 ml liegt;
f) Erhitzen des Reaktionsgemischen, wobei das Olefin zum Methylester umgesetzt wird.

In einer Variante des Verfahrens wird dem Reaktionsgemisch kein CO-Gas zugeführt.

In einer Variante des Verfahrens dient HCOOH als einzige CO-Quelle für die Reaktion.

In einer Variante des Verfahrens ist die Verbindung in Verfahrensschritt b) ausgewählt aus: Pd(acac)₂, PdCl₂, Pd(dba)₃*CH₃Cl (dba = Dibenzylidenaceton), Pd(OAc)₂, Pd(TFA)₂, Pd(CH₃CN)Cl₂.

In einer Variante des Verfahrens ist die Verbindung in Verfahrensschritt b) Pd(OAc)₂.

In einer Variante des Verfahrens umfasst das Verfahren den zusätzlichen Verfahrensschritt g): g) Zugabe einer Säure.

In einer Variante des Verfahrens ist die Säure ausgewählt aus: H₂SO₄, CH₃SO₃H, CF₃SO₃H, PTSA (p-Toluolsulfonsäure).

In einer Variante des Verfahrens ist die Säure PTSA (p-Toluolsulfonsäure).

In einer Variante des Verfahrens liegt das eingesetzte Volumen an HCOOH bezogen auf 2 mmol Olefin im Bereich von 0,4 ml bis 0,6 ml.

In einer Variante des Verfahrens sind R¹, R², R³, R⁴ jeweils unabhängig voneinander ausgewählt aus: -(C₁-C₁₂)-Alkyl, -O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, - COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

In einer Variante des Verfahrens sind R¹, R², R³, R⁴ jeweils unabhängig voneinander ausgewählt aus: -(C₁-C₁₂)-Alkyl, -(C₄-C₁₄)-Aryl, Cycloalkyl, -(C₁-C₁₂)-Heteroalkyl, -(C₃-C₁₄)-Heteroaryl, Halogen und die Reste auch zur Bildung eines größeren kondensierten Ringes befähigt sind; wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

In einer Variante des Verfahrens sind R¹, R², R³, R⁴ jeweils unabhängig voneinander ausgewählt aus: -(C₁-C₁₂)-Alkyl, Cycloalkyl, -(C₃-C₁₄)-Heteroaryl und die Reste auch zur Bildung eines größeren kondensierten Ringes befähigt sind;
wobei die genannten Alkylgruppen, Cycloalkyl, Heteroarylgruppen wie folgt substituiert sein können:
-(C₁-C₁₂)-Alkyl, -O-(C₁-C₁₂)-Alkyl, Halogen,
und mindestens einer der Reste R¹, R², R³, R⁴ nicht für Phenyl steht.

In einer Variante des Verfahrens sind R¹, R⁴ jeweils unabhängig voneinander ausgewählt aus: - (C₁-C₁₂)-Alkyl, Cycloalkyl und die Reste auch zur Bildung eines größeren kondensierten Ringes befähigt sind;
wobei die genannten Alkylgruppen, Cycloalkyl wie folgt substituiert sein können:
-(C₁-C₁₂)-Alkyl, -O-(C₁-C₁₂)-Alkyl, Halogen.

In einer Variante des Verfahrens stehen R², R³ jeweils unabhängig voneinander für -(C₃-C₁₄)-Heteroaryl,
wobei die genannten Heteroarylgruppen wie folgt substituiert sein können:
-(C₁-C₁₂)-Alkyl, -O-(C₁-C₁₂)-Alkyl, Halogen.

In einer Variante des Verfahrens weist die Verbindung der allgemeinen Formel (**I**) die Struktur (**II**) auf:

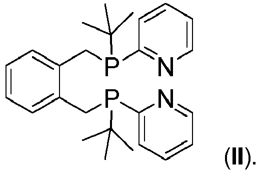

Im Folgenden wird die Erfindung anhand von Ausführungsbeispielen näher erläutert.

### Pd-katalysierte Methoxycarbonylierung von Tetramethylethylen 1a mit HCOOH: Einfluss des eingesetzten Volumens von HCOOH

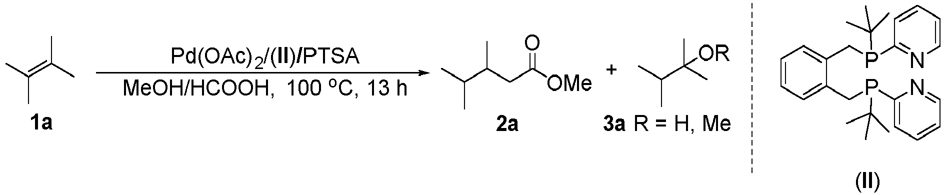

In ein verschlossenes 35-ml-Röhrchen wurden [Pd(OAc)₂] (1,12 mg, 0,25 Mol-%), (**II**) (8,72 mg, 1,0 Mol-%), p-Toluolsulfonsäure (PTSA▪H₂O) (15,2 mg, 4 Mol-%) und ein ofengetrocknetes Rührstäbchen gegeben. Das Röhrchen wurde zusammen mit dem Deckel in ein langes Schlenk-Rohr mit großer Öffnung gegeben. Das Schlenk-Rohr wird dreimal evakuiert und wieder mit Argon aufgefüllt. Unter einer Argonatmosphäre wurden **1a** (2 mmol), MeOH (1,5 ml) und HCOOH (X ml) (X siehe Tabelle 1) mittels einer Spritze in das 35-ml-Röhrchen injiziert. Das 35-ml-Röhrchen wurde dann mit dem Deckel verschlossen. Die Reaktion erfolgte über 13 h bei 100 °C. Nach dem Ende der Umsetzung wurde das Röhrchen ohne zusätzliche Kühlung auf Raumtemperatur kommen gelassen und es wurde vorsichtig entspannt. Dann wurde Isooctan (100 µl) als innerer Standard injiziert. Der Umsatz wurde durch GC-Analyse gemessen.

In der folgenden Tabelle 1 sind die Ergebnisse zusammengefasst:

**Tabelle 1:**

| HCOOH (Volumen in ml) | Umsatz % | **2a** Ausbeute % | **3a** Ausbeute % |
|---|---|---|---|
| 0.2 | 73 | 53 | 17 |
| 0.3 | 85 | 72 | 11 |
| 0.5 | 91 | 80 | 7 |
| 0.8 | 90 | 71 | 5 |

Wie die oben beschriebenen Versuche zeigen, wird die Aufgabe durch ein erfindungsgemäßes Verfahren gelöst.

## Patentansprüche

1. Verfahren umfassend die Verfahrensschritte:
a) Zugabe eines Olefins;
b) Zugabe einer Verbindung, welche Pd umfasst, wobei das Pd in der Lage ist einen Komplex auszubilden;
c) Zugabe einer Verbindung der allgemeinen Formel (**I**): 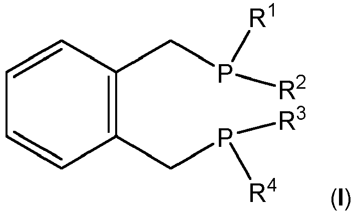 wobei R¹, R², R³, R⁴ jeweils unabhängig voneinander ausgewählt sind aus: -H, -(C₁-C₁₂)-Alkyl, - O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht;
d) Zugabe von MeOH;
e) Zugabe von HCOOH,
wobei das eingesetzte Volumen bezogen auf 2 mmol Olefin im Bereich von 0,3 ml bis 0,8 ml liegt;
f) Erhitzen des Reaktionsgemischen, wobei das Olefin zum Methylester umgesetzt wird.

2. Verfahren nach Anspruch 1,
wobei dem Reaktionsgemisch kein CO-Gas zugeführt wird.

3. Verfahren nach Anspruch 1 oder 2,
wobei HCOOH als einzige CO-Quelle für die Reaktion dient.

4. Verfahren nach einem der Ansprüche 1 bis 3,
wobei die Verbindung in Verfahrensschritt b) ausgewählt ist aus:
Pd(acac)₂, PdCl₂, Pd(dba)₃*CH₃Cl (dba = Dibenzylidenaceton), Pd(OAc)₂, Pd(TFA)₂, Pd(CH₃CN)Cl₂.

5. Verfahren nach einem der Ansprüche 1 bis 4,
wobei das Verfahren den zusätzlichen Verfahrensschritt g) umfasst:
g) Zugabe einer Säure.

6. Verfahren nach Anspruch 5,
wobei die Säure ausgewählt ist aus: H₂SO₄, CH₃SO₃H, CF₃SO₃H, PTSA.

7. Verfahren nach einem der Ansprüche 1 bis 6,
wobei das eingesetzte Volumen an HCOOH bezogen auf 2 mmol Olefin im Bereich von 0,4 ml bis 0,6 ml liegt.

8. Verfahren nach einem der Ansprüche 1 bis 7,
wobei R¹, R², R³, R⁴ jeweils unabhängig voneinander ausgewählt sind aus: -(C₁-C₁₂)-Alkyl, -O-(C₁-C₁₂)-Alkyl, -(C₄-C₁₄)-Aryl, -O-(C₄-C₁₄)-Aryl, Cycloalkyl, -(C₁-C₁₂)-Heteroalkyl, -O-(C₁-C₁₂)-Heteroalkyl, -(C₃-C₁₄)-Heteroaryl, -O-(C₃-C₁₄)-Heteroaryl, -COO-Alkyl, -COO-Aryl, -C-O-Alkyl, -C-O-Aryl, NH₂, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

9. Verfahren nach einem der Ansprüche 1 bis 8,
wobei R¹, R², R³, R⁴ jeweils unabhängig voneinander ausgewählt sind aus: -(C₁-C₁₂)-Alkyl, -(C₄-C₁₄)-Aryl, Cycloalkyl, -(C₁-C₁₂)-Heteroalkyl, -(C₃-C₁₄)-Heteroaryl, Halogen und die Reste auch zur Bildung eines größeren kondensierten Ringes befähigt sind;
wobei die genannten Alkylgruppen, Arylgruppen, Cycloalkyl, Heteroalkylgruppen, Heteroarylgruppen wie folgt substituiert sein können:
-(C₁-C₁₂)-Alkyl, -O-(C₁-C₁₂)-Alkyl, Halogen;
und mindestens einer der Reste R¹, R², R³, R⁴ nicht für Phenyl steht.

10. Verfahren nach einem der Ansprüche 1 bis 9,
wobei R¹, R², R³, R⁴ jeweils unabhängig voneinander ausgewählt sind aus: -(C₁-C₁₂)-Alkyl, Cycloalkyl, -(C₃-C₁₄)-Heteroaryl und die Reste auch zur Bildung eines größeren kondensierten Ringes befähigt sind;
wobei die genannten Alkylgruppen, Cycloalkyl, Heteroarylgruppen wie folgt substituiert sein können:
-(C₁-C₁₂)-Alkyl, -O-(C₁-C₁₂)-Alkyl, Halogen,
und mindestens einer der Reste R¹, R², R³, R⁴ nicht für Phenyl steht.

11. Verfahren nach einem der Ansprüche 1 bis 10,
wobei R¹, R⁴ jeweils unabhängig voneinander ausgewählt sind aus: -(C₁-C₁₂)-Alkyl, Cycloalkyl und die Reste auch zur Bildung eines größeren kondensierten Ringes befähigt sind;
wobei die genannten Alkylgruppen, Cycloalkyl wie folgt substituiert sein können:
-(C₁-C₁₂)-Alkyl, -O-(C₁-C₁₂)-Alkyl, Halogen.

12. Verfahren nach einem der Ansprüche 1 bis 11,
wobei R², R³ jeweils unabhängig voneinander für -(C₃-C₁₄)-Heteroaryl stehen,
wobei die genannten Heteroarylgruppen wie folgt substituiert sein können:
-(C₁-C₁₂)-Alkyl, -O-(C₁-C₁₂)-Alkyl, Halogen.

13. Verfahren nach einem der Ansprüche 1 bis 12,
wobei die Verbindung der allgemeinen Formel (**I**) die Struktur (**II**) aufweist:

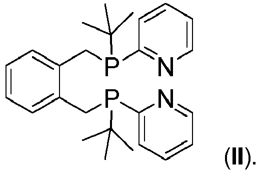

## Claims

1. Process comprising the process steps of:
a) addition of an olefin;
b) addition of a compound comprising Pd, wherein the Pd is capable of forming a complex;
c) addition of a compound of general formula (**I**): 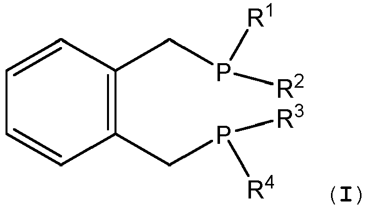 wherein R¹, R², R³ and R⁴ are each independently selected from: -H, -(C₁-C₁₂)-alkyl, -O-(C₁-C₁₂)-alkyl, -(C₄-C₁₄)-aryl, -O-(C₄-C₁₄)-aryl, cycloalkyl, -(C₁-C₁₂)-heteroalkyl, -O-(C₁-C₁₂)-heteroalkyl, -(C₃-C₁₄)-heteroaryl, -O-(C₃-C₁₄)-heteroaryl, -COO-alkyl, -COO-aryl, -C-O-alkyl, -C-O-aryl, NH₂, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl;
d) addition of MeOH;
e) addition of HCOOH,
wherein the employed volume based on 2 mmol of olefin is in the range from 0.3 ml to 0.8 ml;
f) heating of the reaction mixture to convert the olefin into the methyl ester.

2. Process according to Claim 1,
wherein no CO gas is supplied to the reaction mixture.

3. Process according to Claim 1 or 2,
wherein HCOOH serves as the only CO source for the reaction.

4. Process according to any of Claims 1 to 3,
wherein the compound in process step b) is selected from:
Pd(acac)₂, PdCl₂, Pd(dba)₃*CH₃Cl (dba = dibenzylideneacetone), Pd(OAc)₂, Pd(TFA)₂, Pd(CH₃CN)Cl₂.

5. Process according to any of Claims 1 to 4,
wherein the process comprises the additional process step g):
g) addition of an acid.

6. Process according to Claim 5,
wherein the acid is selected from: H₂SO₄, CH₃SO₃H, CF₃SO₃H, PTSA.

7. Process according to any of Claims 1 to 6,
wherein the employed volume of HCOOH based on 2 mmol of olefin is in the range from 0.4 ml to 0.6 ml.

8. Process according to any of Claims 1 to 7,
wherein R¹, R², R³ and R⁴ are each independently selected from: -(C₁-C₁₂)-alkyl, -O-(C₁-C₁₂)-alkyl, -(C₄-C₁₄)-aryl, - O-(C₄-C₁₄)-aryl, cycloalkyl, -(C₁-C₁₂)-heteroalkyl, -O-(C₁-C₁₂)-heteroalkyl, -(C₃-C₁₄)-heteroaryl, -O-(C₃-C₁₄)-heteroaryl, -COO-alkyl, -COO-aryl, -C-O-alkyl, -C-O-aryl, NH₂, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl.

9. Process according to any of Claims 1 to 8,
wherein R¹, R², R³ and R⁴ are each independently selected from: -(C₁-C₁₂)-alkyl, -(C₄-C₁₄)-aryl, cycloalkyl, -(C₁-C₁₂) -heteroalkyl, -(C₃-C₁₄)-heteroaryl, halogen and the residues are also capable of forming a larger condensed ring;
wherein the recited alkyl groups, aryl groups, cycloalkyl, heteroalkyl groups, heteroaryl groups may be substituted as follows:
-(C₁-C₁₂)-alkyl, -O-(C₁-C₁₂)-alkyl, halogen;
and at least one of the radicals R¹, R², R³, R⁴ does not represent phenyl.

10. Process according to any of Claims 1 to 9,
wherein R¹, R², R³ and R⁴ are each independently selected from: -(C₁-C₁₂)-alkyl, cycloalkyl, -(C₃-C₁₄)-heteroaryl and the residues are also capable of forming a larger condensed ring;
wherein the recited alkyl groups, cycloalkyl, heteroaryl groups may be substituted as follows:
-(C₁-C₁₂)-alkyl, -O-(C₁-C₁₂)-alkyl, halogen,
and at least one of the radicals R¹, R², R³, R⁴ does not represent phenyl.

11. Process according to any of Claims 1 to 10,
wherein R¹, R⁴ are each independently selected from: - (C₁-C₁₂)-alkyl, cycloalkyl, and the residues are also capable of forming a larger condensed ring;
wherein the recited alkyl groups, cycloalkyl may be substituted as follows:
-(C₁-C₁₂)-alkyl, -O-(C₁-C₁₂)-alkyl, halogen.

12. Process according to any of Claims 1 to 11,
wherein R², R³ each independently represent -(C₃-C₁₄)-heteroaryl,
wherein the recited heteroaryl groups may be substituted as follows:
-(C₁-C₁₂)-alkyl, -O-(C₁-C₁₂)-alkyl, halogen.

13. Process according to any of Claims 1 to 12,
wherein the compound of general formula (**I**) has the structure (**II**):

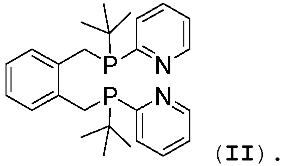

## Revendications

1. Procédé comprenant les étapes de procédé suivantes :
a) ajout d'une oléfine ;
b) ajout d'un composé, qui comprend du Pd, le Pd étant en état de former un complexe ;
c) ajout d'un composé de formule générale (I) : 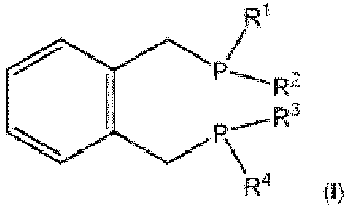 R¹, R², R³, R⁴ à chaque fois indépendamment les uns des autres étant choisis parmi : -H, -C₁₋₁₂-alkyle, -O-C₁₋₁₂-alkyle, -C₄₋₁₄-aryle, -O-C₄₋₁₄-aryle, cycloalkyle, -C₁₋₁₂-hétéroalkyle, -OC₁₋₁₂-hétéroalkyle, -C₃₋₁₄-hétéroaryle, -O-C₃₋₁₄-hétéroaryle, -COO-alkyle, -COO-aryle, -C-O-alkyle, -C-O-aryle, NH₂, halogène et les radicaux étant aussi aptes à la formation d'un cycle condensé plus grand ;
les groupes alkyle, les groupes aryle, cycloalkyle, les groupes hétéroalkyle, les groupes hétéroaryle mentionnés pouvant être substitués comme suit :
-C₁₋₁₂-alkyle, -O-C₁₋₁₂-alkyle, halogène ;
et au moins un des radicaux R¹, R², R³, R⁴ ne représentant pas phényle ;
d) ajout de MeOH;
e) ajout de HCOOH,
le volume utilisé, par rapport à 2 mmol d'oléfine, se situant dans la plage de 0,3 ml à 0,8 ml ;
f) chauffage du mélange réactionnel, l'oléfine étant transformée en un ester méthylique.

2. Procédé selon la revendication 1,
aucun gaz de CO n'étant introduit dans le mélange réactionnel.

3. Procédé selon la revendication 1 ou 2,
HCOOH servant de seule source de CO pour la réaction.

4. Procédé selon l'une quelconque des revendications 1 à 3,
le composé dans l'étape de procédé b) étant choisi parmi :
Pd(acac)₂, PdCl₂, Pd(dba)₃*CH₃Cl (dba = dibenzylidèneacétone), Pd(OAc)₂, Pd(TFA)₂, Pd(CH₃CN)Cl₂.

5. Procédé selon l'une quelconque des revendications 1 à 4,
le procédé comprenant l'étape de procédé supplémentaire g) :
g) ajout d'un acide.

6. Procédé selon la revendication 5,
l'acide étant choisi parmi : H₂SO₄, CH₃SO₃H, CF₃SO₃H, PTSA.

7. Procédé selon l'une quelconque des revendications 1 à 6,
le volume utilisé de HCOOH, par rapport à 2 mmol d'oléfine, se situant dans la plage de 0,4 ml à 0,6 ml.

8. Procédé selon l'une quelconque des revendications 1 à 7,
R¹, R², R³, R⁴ à chaque fois indépendamment les uns des autres étant choisis parmi : -C₁₋₁₂-alkyle, -OC₁₋₁₂-alkyle, -C₄₋₁₄-aryle, -O-C₄₋₁₄-aryle, cycloalkyle, -C₁₋₁₂-hétéroalkyle, -O-C₁₋₁₂-hétéroalkyle, -C₃₋₁₄-hétéroaryle, -O-C₃₋₁₄-hétéroaryle, -COO-alkyle, -COO-aryle, -C-O-alkyle, -C-O-aryle, NH₂, halogène et les radicaux étant aussi aptes à la formation d'un cycle condensé plus grand ;
les groupes alkyle, les groupes aryle, cycloalkyle, les groupes hétéroalkyle, les groupes hétéroaryle mentionnés pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

9. Procédé selon l'une quelconque des revendications 1 à 8,
R¹, R², R³, R⁴ à chaque fois indépendamment les uns des autres étant choisis parmi : -C₁₋₁₂-alkyle, -C₄₋₁₄-aryle, cycloalkyle, -C₁₋₁₂-hétéroalkyle, -C₃₋₁₄-hétéroaryle, halogène et les radicaux étant aussi aptes à la formation d'un cycle condensé plus grand ;
les groupes alkyle, les groupes aryle, cycloalkyle, les groupes hétéroalkyle, les groupes hétéroaryle mentionnés pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

10. Procédé selon l'une quelconque des revendications 1 à 9,
R¹, R², R³, R⁴ à chaque fois indépendamment les uns des autres étant choisis parmi : -C₁₋₁₂-alkyle, cycloalkyle, -C₃₋₁₄-hétéroaryle et les radicaux étant aussi aptes à la formation d'un cycle condensé plus grand ;
les groupes alkyle, cycloalkyle, les groupes hétéroaryle mentionnés pouvant être substitués comme suit :
-C₁₋₁₂-alkyle, -O-C₁₋₁₂-alkyle, halogène ;
et au moins un des radicaux R¹, R², R³, R⁴ ne représentant pas phényle.

11. Procédé selon l'une quelconque des revendications 1 à 10,
R¹, R⁴ à chaque fois indépendamment l'un de l'autre étant choisis parmi : -C₁₋₁₂-alkyle, cycloalkyle et les radicaux étant aussi aptes à la formation d'un cycle condensé plus grand ;
les groupes alkyle, cycloalkyle mentionnés pouvant être substitués comme suit :
-C₁₋₁₂-alkyle, -O-C₁₋₁₂-alkyle, halogène.

12. Procédé selon l'une quelconque des revendications 1 à 11,
R², R³ à chaque fois indépendamment l'un de l'autre représentant -C₃₋₁₄-hétéroaryle,
les groupes mentionnés hétéroaryle pouvant être substitués comme suit :
-C₁₋₁₂-alkyle, -O-C₁₋₁₂-alkyle, halogène.

13. Procédé selon l'une quelconque des revendications 1 à 12,
le composé de formule générale (I) présentant la structure (II) :
